# EUROPÄISCHE PATENTSCHRIFT

(11) **EP 1 658 024 B1**
(45) Veröffentlichungstag und Bekanntmachung des Hinweises auf die Patenterteilung: **22.11.2006**
(21) Anmeldenummer: 04763651.9
(22) Anmeldetag: 30.07.2004
(51) Int. Cl.: A61F 2/44, A61F 2/46

(54) **IMPLANTAT ZUM VERSCHLUSS EINER ÖFFNUNG DES ANNULUS FIBROSUS**
IMPLANT FOR CLOSING AN OPENING OF THE ANNULUS FIBROSUS
IMPLANT DESTIN FERMER UNE OUVERTURE DANS L'ANNEAU FIBREUX

(30) Priorität: 26.08.2003 DE 10340150
(43) Veröffentlichungstag der Anmeldung: 24.05.2006
(73) Patentinhaber: Aesculap AG & Co. KG, 78532 Tuttlingen (DE)
(72) Erfinder: BLÖMER, Wilhelm, 88690 Unteruhldingen-Mühlhofen (DE); SCHULTZ, Robert, 78532 Tuttlingen (DE); MAYER, Michael, 82166 Gräfelfing (DE)
(74) Vertreter: Boehme, Ulrich
(86) Internationale Anmeldenummer: PCT/EP2004/008567
(87) Internationale Veröffentlichungsnummer: WO 2005/020859

(56) Entgegenhaltungen:
- WO-A-02/058599
- US-A1- 2003 078 579
- US-A1- 2003 195 514

## Beschreibung

Die Erfindung betrifft ein Implantat zum Verschluß einer Öffnung des Annulus fibrosus in Form eines Stopfens.

Die menschliche Wirbelsäule, insbesondere die Bandscheiben, sind einem natürlichen Alterungsprozeß unterworfen. Dabei verringert sich der Flüssigkeitsgehalt des gelartigen Kerns der Bandscheibe (Nucleus pulposus). Dadurch verliert die Bandscheibe an Höhe, und der faserartige Ring, der den Nucleus umgibt (Annulus fibrosus), wird immer stärker beansprucht. Es können Risse im Annulus entstehen, durch welche Nucleusmaterial nach außen gedrückt wird. So entstehen zunächst Vorwölbungen (Prolaps), bei denen das Nucleusmaterial noch innerhalb der Bandscheibe bleibt. Ist das Nucleusmaterial vollständig aus dem Annulus ausgetreten, spricht man von einem Sequester. Prolaps und Sequester können Schmerzen von unterschiedlichem Ausmaß hervorrufen, je nachdem wie stark die benachbarten Nervenwurzeln oder der Spinalkanal beeinträchtigt werden.

Bei einer Bandscheibenoperation wird deshalb zunächst alles ausgetretene Nucleusmaterial entfernt. Um sicherzugehen, daß es zu keinem erneuten Bandscheibenvorfall kommt, wird auch ein Teil des noch in der Bandscheibe befindlichen Nucleus entfernt (Nucleotomie). Das dabei entfernte Material kann zur Wiederherstellung oder Regeneration des Nucleus verwendet werden. Es wird nämlich ein Teil des Nucleusmaterials, welches dem Patienten bei der Nucleotomie entnommen wurde, dazu benutzt, um biotechnologisch Zellen neu anzuzüchten. Es wird ein Bandscheibenzelltransplatat aus körpereigenen Zellen hergestellt, welches dem Patienten etwa zwei Wochen nach der Bandscheibenoperation wieder in die Bandscheibe injiziert wird. Die injizierten Zellen siedeln sich wieder an und tragen zu einer Regeneration des noch vorhandenen Nucleus bei.

Allerdings ergibt sich dadurch ein Problem, daß der Annulus nicht mehr intakt ist, durch die bei der Nucleotomie erzeugte Öffnung und durch Risse im Annulus können die injizierten Zellen wieder entweichen und absterben, noch bevor sie anwachsen konnten.

Es ist bekannt, Öffnungen im Annulus durch Einsetzen von Annulusverschlüssen zu verschließen (WO 01/10316 A1; WO 99/04720; WO 02/080821; WO 02/058599 A WO 01/28464). In der Mehrzahl der Fälle stützt sich dabei der verwendete Verschlußkörper direkt am Annulusgewebe ab und ist mit diesem vernäht, verklebt oder in anderer Weise verbunden. Da das Annulusgewebe ein sehr flexibles Gewebe ist, ist auch der Verschluß den Bewegungen des Annulus ausgesetzt, und es besteht die Gefahr, daß dieser Verschluß sich unbeabsichtigt löst. In Ausnahmefällen wird der Verschlußkörper auch an den benachbarten Wirbelkörpern festgelegt, es handelt sich dann aber ausschließlich um netzartige oder membranartige Verschlüsse, die nach Art einer Folie die Annulusöffnung verschließen sollen. Dabei ist eine sichere Abdichtung nicht gewährleistet.

Es ist Aufgabe der Erfindung, ein gattungsgemäßes Implantat so auszubilden, daß einerseits eine sichere Festlegung des Implantates sichergestellt ist und andererseits ein zuverlässiger Verschluß der Annulusöffnung, wobei die Beweglichkeit des Annulus erhalten bleiben soll.

Diese Aufgabe wird bei einem Implantat der eingangs beschriebenen Art erfindungsgemäß dadurch gelöst, daß der Stopfen an seiner Oberseite und an seiner Unterseite formstabile Verankerungselemente für die Endplatten benachbarter Wirbelkörper und im Bereich zwischen Oberseite und Unterseite eine verformbare Struktur aufweist.

Es wird also ein Implantat in Form eines Stopfens verwendet, welcher in die Öffnung des Annulus eingesetzt wird und welcher im wesentlichen eine verformbare Struktur aufweist, so daß er sich an die Struktur des Annulus anpaßt und sich insbesondere im Abdichtungsbereich am Rand der zu verschließenden Öffnung dicht an das Annulusgewebe anlegt, während er andererseits die freie Beweglichkeit des Annulusgewebes nicht beeinträchtigt. Andererseits läßt sich dieser verformbare Stopfen durch die formstabilen Verankerungselemente an der Oberseite und an der Unterseite des Stopfens an den Endplatten der benachbarten Wirbelkörper festlegen, so daß die Wirbelkörper die Positionierung des Stopfens gewährleisten, so daß dieser durch die Bewegung des Annulus nicht aus seiner Position entfernt wird.

Die formstabilen Verankerungselemente können beispielsweise dadurch gebildet werden, daß gemäß einer bevorzugten Ausführungsform die Oberseite und/oder die Unterseite formstabiler sind als der zwischen Oberseite und Unterseite liegende Bereich des Stopfens. In diesem Falle wirken die Oberseite und die Unterseite als Verankerungselemente und bilden Anlageflächen, die an den Endplatten anliegen und dadurch den Stopfen in seiner Lage fixieren.

Es ist günstig, wenn das Implantat aus resorbierbarem Material besteht, so daß sich der Stopfen nach einiger Zeit auflöst. Sobald die implantierten Zellkulturen angewachsen sind, verschließen sie den Annulus wieder, so daß dann der Stopfen durch körpereigenes Gewebe ersetzt werden kann.

Der Stopfen kann insbesondere aus einem porösen Material bestehen, die Porengröße kann beispielsweise zwischen 150 µm und 200 µm liegen. Es ist dadurch möglich, Zellkulturen auch in den Stopfen zu injizieren, so daß diese auch in diesem Bereich anwachsen und die Öffnung beschleunigt verschließen.

Besonders vorteilhaft ist es, wenn die Oberseite und die Unterseite mindestens einen in die Endplatte des benachbarten Wirbel körpers einsetzbaren Vorsprung tragen. Diese Vorsprünge können in das Material der benachbarten Wirbelkörper eingreifen und fixieren dadurch den Stopfen in der gewünschten Lage. Beispielsweise ist es möglich, im Wirbelkörper entsprechende Vertiefungen auszuarbeiten, in die diese Vorsprünge eingreifen.

Günstig ist es, wenn mehrere Vorsprünge in einer Linie nebeneinander liegen, diese Vorsprünge können dann beispielsweise in eine linienförmige Vertiefung der Endplatte eingreifen.

Bei einer anderen Ausführungsform ist vorgesehen, daß der Vorsprung eine Längsrippe ist.

Dabei ist es vorteilhaft, wenn die Linie der Vorsprünge bzw. die Längsrichtung der Längsrippe parallel zur Einschubrichtung des Stopfens verläuft. Man kann auf diese Weise den Stopfen in die Öffnung einschieben, und gleichzeitig greifen die in einer Linie hintereinander angeordneten Vorsprünge oder die entsprechend ausgerichtete Längsrippe in eine in Vorschubrichtung verlaufende Rinne im Wirbelkörper ein, die vor dem Einsetzen in den Wirbelkörper eingearbeitet wird. Man erhält auf diese Weise eine sehr sichere Festlegung des Stopfens.

Eine zusätzliche Fixierung des Stopfens kann man dadurch erreichen, daß der Vorsprung eine Zahnung aufweist.

Insbesondere bei größeren Stopfen ist es günstig, wenn quer zur Einschubrichtung des Stopfens nebeneinander mehrere Vorsprünge angeordnet sind, so daß eine besonders homogene Festlegung des Stopfens in den benachbarten Wirbelkörpern erfolgen kann.

Dabei ist es vorteilhaft, wenn die Vorsprünge gegenüber der Seitenfläche des Stopfens zurückgesetzt sind, so daß die seitlich überstehenden Teile des Stopfens sich optimal an das Gewebe des Annulus anpassen können, da dieses Gewebe flexibel und gegebenenfalls elastisch ausgebildet ist.

Bei einer bevorzugten Ausführungsform ist vorgesehen, daß der Stopfen die Form eines Würfels oder Quaders hat, es sind aber auch andere Formen des Stopfens möglich, beispielsweise können Stopfen in Zylinderform verwendet werden, deren Längsachse parallel zur Einschubrichtung verläuft.

Bei einer besonders bevorzugten Ausführungsform ist der Stopfen mit einer Membran abgedeckt, die ebenfalls aus resorbierbarem Material besteht, dies dichtet den Stopfen nach außen zusätzlich ab, so daß in ihn injizierte Zellen sicher im Stopfen verbleiben.

Günstig ist es dabei, wenn die Membran langsamer resorbierbar ist als der Stopfen.

Weiterhin kann vorgesehen sein, daß der Stopfen an seiner Außenseite mit einem Anti-Adhäsionsmittel versehen ist, um in diesem Bereich die Bildung von Narbengewebe zu vermeiden.

Es ist vorteilhaft, wenn das Implantat Teil eines Satzes von Stopfen mit unterschiedlicher Höhe und/oder Breite ist, so daß der Operateur während der Operation aus diesem Satz einen Stopfen der geeigneten Abmessungen auswählen kann und dadurch eine optimale Abdichtung der Öffnung des Annulus fibrosus erreicht.

Die nachfolgende Beschreibung bevorzugter Ausführungsformen der Erfindung dient im Zusammenhang mit der Zeichnung der näheren Erläuterung. Es zeigen:
- Figur 1a:: eine schematische Draufsicht auf den zwischen zwei Wirbelkörpern angeordneten Annulus fibrosus mit einem eingesetzten Implantat zum Verschluß einer Öffnung in diesem;
- Figur 1b:: eine Seitenansicht auf die Wirbelkörper der Figur 1;
- Figur 2:: eine perspektivische Ansicht des Implantates der Figuren 1a und 1b mit geänderten Abmessungen;
- Figur 3:: ein abgewandeltes Ausführungsbeispiel eines Implantates mit mehreren Stegen zur Verankerung in den benachbarten Wirbelkörpern;
- Figur 4:: eine schematische Ansicht eines Wirbelkörpers und eines Annulus fibrosus mit einem Bandscheibenvorfall;
- Figur 5:: eine Darstellung ähnlich Figur 4 beim Eröffnen des Annulus fibrosus und beim Entfernen von überschüssigem Nucleusgewebe;
- Figur 6:: eine Ansicht ähnlich Figur 1 beim Einsetzen eines Probeimplantats in die Annulusöffnung;
- Figur 7:: eine Ansicht ähnlich Figur 6 beim Einsetzen eines Meißels zur Vorbereitung der Endplatten des benachbarten Wirbelkörpers;
- Figur 8:: eine Ansicht ähnlich Figur 1 beim Einsetzen eines Implantates zum Verschluß des Annulus;
- Figur 9:: eine Ansicht ähnlich Figur 4 mit eingesetztem Implantat und
- Figur 10:: eine Ansicht ähnlich Figur 9 beim Injizieren von Zellmaterial in das Implantat und in den Nucleus.

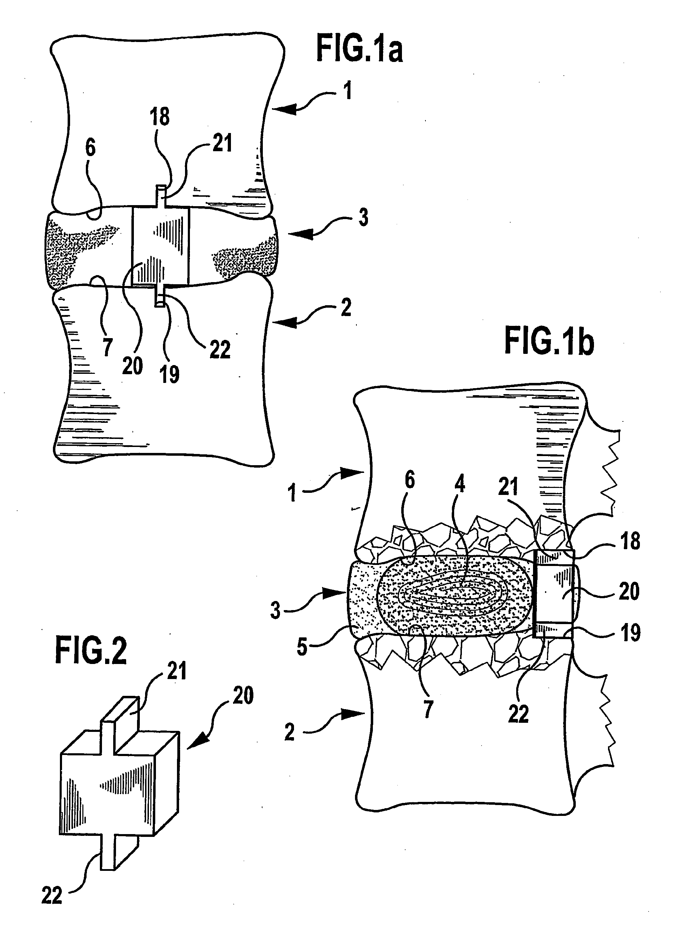

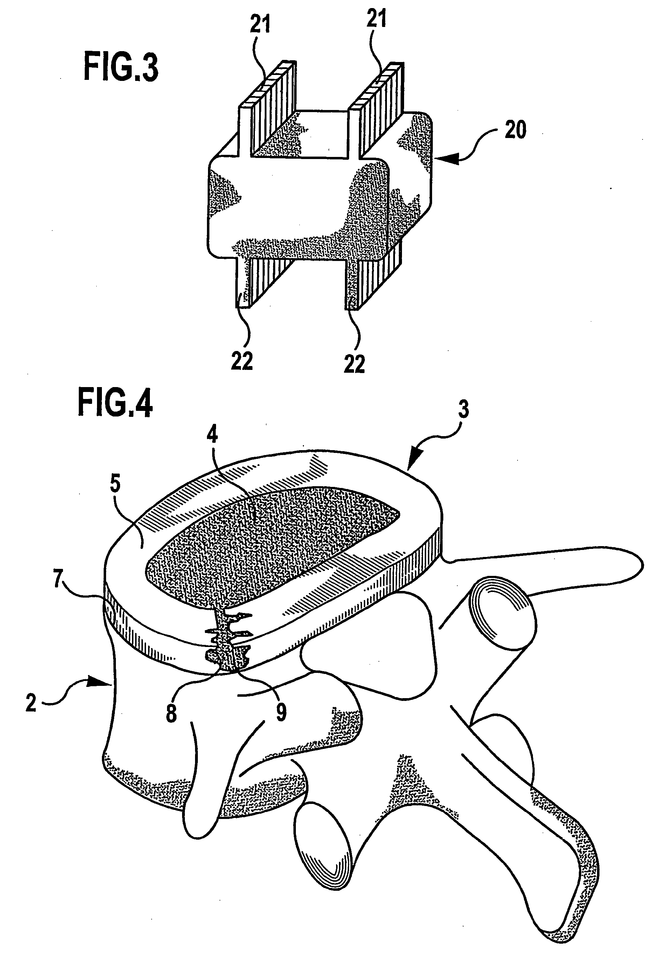

Wie aus der Zeichnung, insbesondere aus den Figuren 1a, 1b und 4 deutlich wird, ist zwischen zwei Wirbelkörpern 1, 2 eine Bandscheibe 3 angeordnet, die aus dem zentralen Nucleus 4 und dem sie umgebenden Annulus fibrosus 5 besteht. In der Darstellung der Figur 4 ist der obere Wirbelkörper 1 der besseren Sichtbarkeit wegen weggelassen, es versteht sich aber, daß dieser obere Wirbelkörper 1 mit seiner unteren Endplatte 6 ebenso wie der untere Wirbelkörper 2 mit seiner oberen Endplatte 7 flächig an der Bandscheibe 3 anliegt.

In Figur 4 ist schematisch dargestellt, daß durch eine Öffnung 8 im Annulus fibrosus, die beispielsweise die Form eines Risses haben kann, Nucleusmaterial 9 aus dem Nucleus 4 nach außen austritt, dieses austretende Material kann auf benachbarte Nervenwurzeln drücken und Schmerzen verursachen.

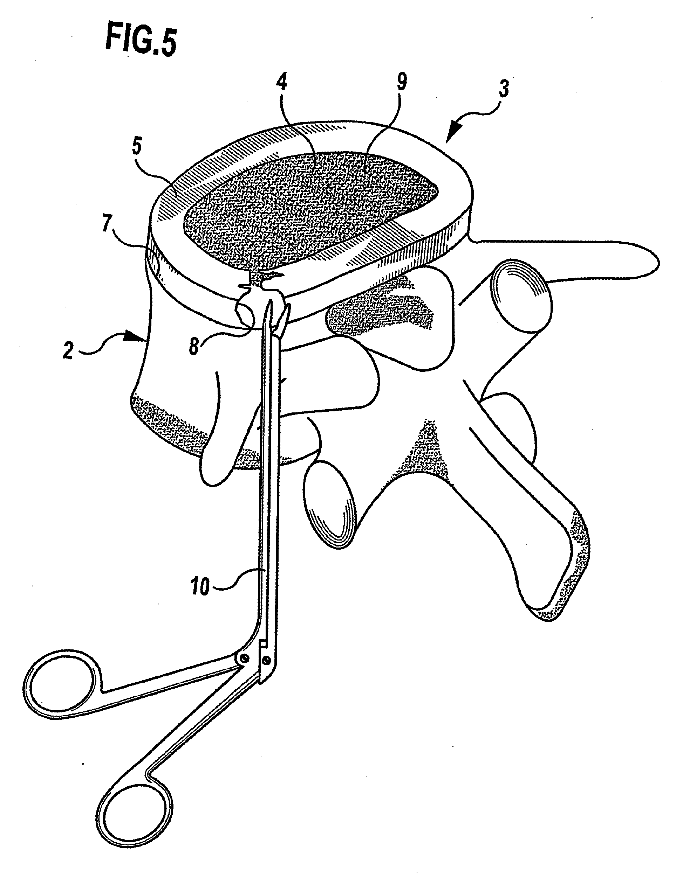

Um diese Verletzung zu beheben, wird in einer Operation das aus der Öffnung 8 austretende Nucleusmaterial 9 entfernt, dies kann beispielsweise mit Hilfe eines zangenförmigen Instrumentes 10 erfolgen, welches das gallertartige Nucleusmaterial 9 abtrennt und auch dazu verwendet werden kann, die Öffnung 8 zu vergrößern bzw. an den Rändern zu glätten (Figur 5). Das dabei entfernte Nucleusmaterial 9 kann zur Anzucht einer körpereigenen Zellkultur verwendet werden, auf diese Weise gelingt es, beispielsweise innerhalb von zwei Wochen eine Zellkultur herzustellen, die dann in der nachstehend beschriebenen Weise verwendet werden kann.

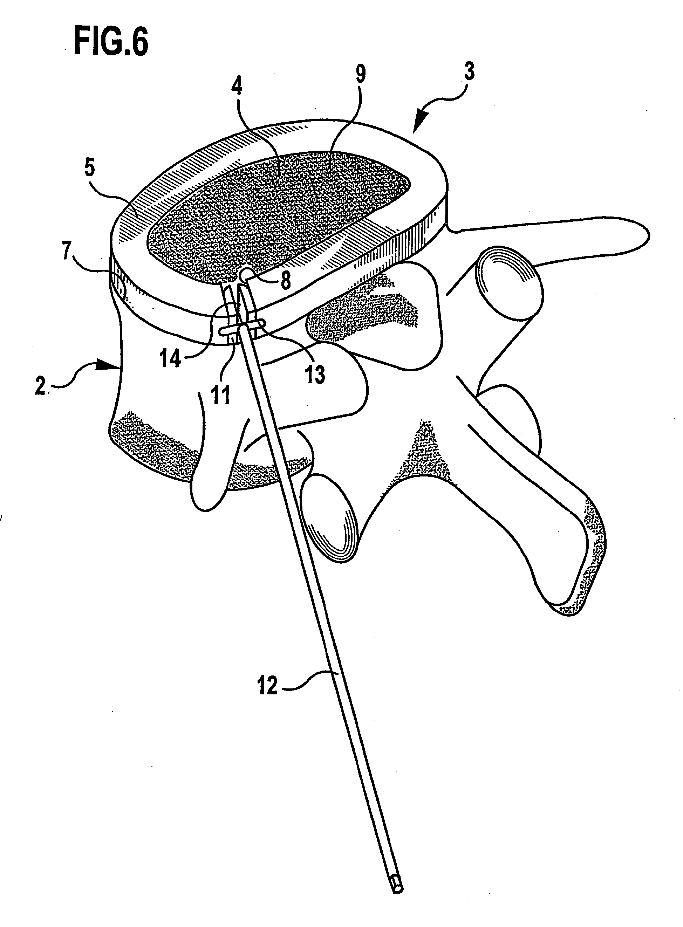

In einem weiteren Operationsschritt muß die Öffnung 8 verschlossen werden. Dazu wird zunächst in die Öffnung 8 ein Probeimplantat 11 eingesetzt, welches beispielsweise aus einem körperverträglichen Metall besteht und im Verhältnis zur Größe der Öffnung 8 so gewählt wird, daß beim Einführen des Probeimplantates 11 in die Öffnung 8 diese nicht unnötig vergrößert wird. Das Probeimplantat 11 kann beispielsweise die Form eines relativ schmalen Quaders haben. Das Probeimplantat 11 ist über eine Stange 12 mit einem in der Zeichnung nicht dargestellten Handgriff verbindbar (Figur 6) und weist seitliche Vorsprünge 13 auf, die die Einschubtiefe in die Öffnung 8 begrenzen.

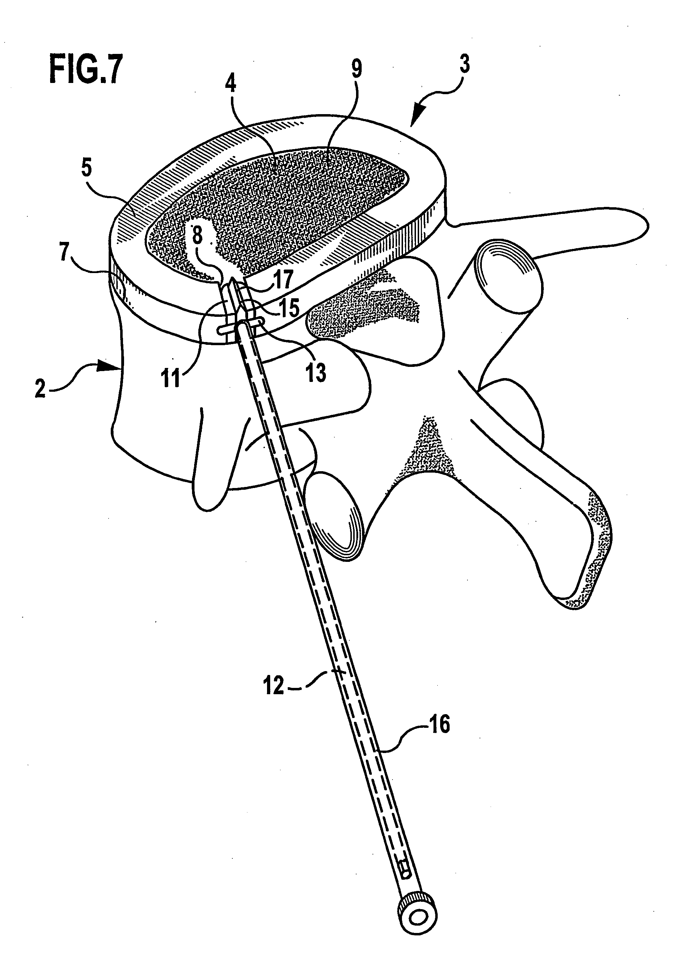

An seiner Oberseite und an seiner Unterseite ist in das Probeimplantat 11 eine zur Stange 12 parallele, mittige Nut 14 eingearbeitet, diese bildet eine Führungsnut aus für einen Meißel 15. Dieser Meißel 15 ist am Ende eines rohrförmigen Schaftes 16 gehalten, der nach Abnahme des Handgriffes von der Stange 12 über diese geschoben werden kann (Figur 7). Dabei wird der Meißel 15 in der Nut 14 geführt und steht mit seiner Schneide 17 aus dieser hervor. Wird der Meißel 15 längs der Stange 12 vorgetrieben, schneidet er mit seiner Schneide 17 eine Nut 18 in die benachbarte Endplatte 6 des Wirbelkörpers 1, diese Nut verläuft dann parallel zur Nut 14 im Probeimplantat 11.

In gleicher Weise kann auf der Unterseite des Probeimplantats 11 vorgegangen werden, auf diese Weise wird dort eine entsprechende Nut 19 in die Endplatte 7 des Wirbelkörpers 2 eingearbeitet.

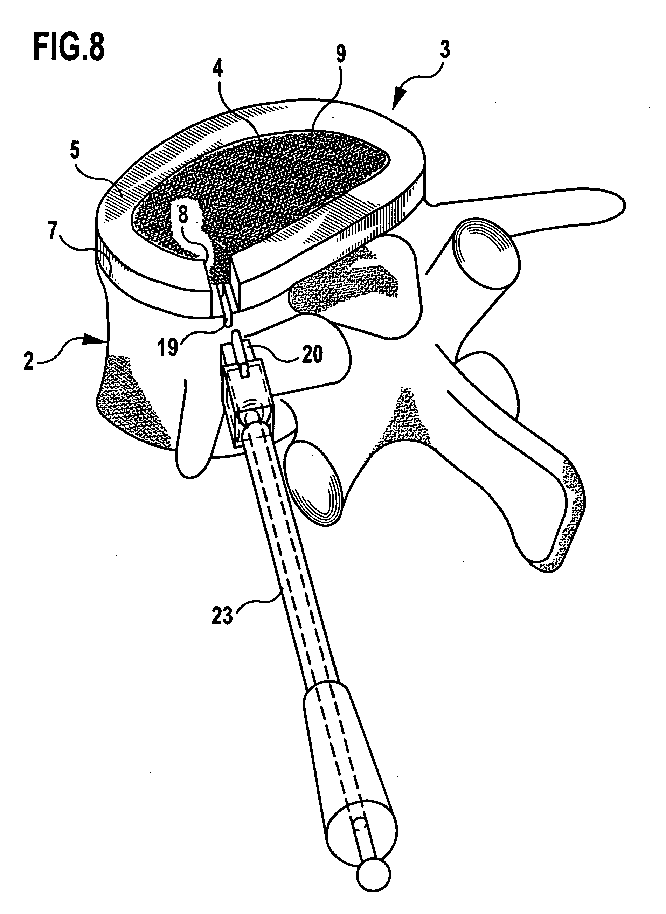

Nach Entnahme des Probeimplantates 11 kann in die Öffnung 8 nunmehr ein diese verschließendes Implantat 20 eingesetzt werden, welches in dem in Figur 8 dargestellten Ausführungsbeispiel die Form eines Quaders aufweist, der an seiner Oberseite und an seiner Unterseite mittig verlaufende Stege oder Rippen 21, 22 trägt, die parallel zu den Kanten des Implantates 20 verlaufen. Das Implantat 20 wird mittels eines geeigneten, das Implantat 20 lösbar ergreifenden Einsetzinstrumentes 23 in die Öffnung 8 eingeschoben, die Vorschubrichtung verläuft dabei parallel zur Längsrichtung der Rippen 21, 22, die beim Einschieben in die Nuten 18 bzw. 19 eintreten. Sobald das Implantat 20 vollständig in die Öffnung 8 eingeschoben ist, kann das Einsetzinstrument gelöst und abgenommen werden.

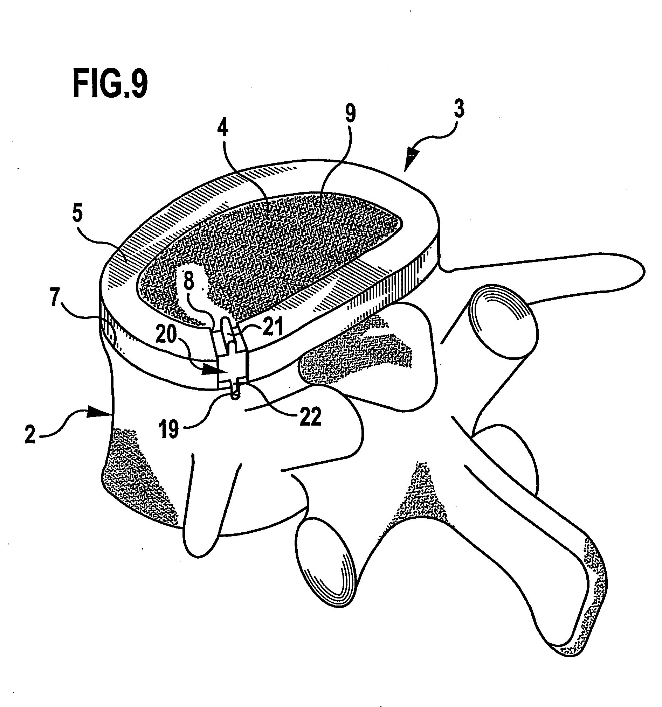

Wie aus der Darstellung der Figur 9 erkennbar ist, verschließt nunmehr das Implantat 20 die Öffnung 8 und stützt sich mit seiner Oberseite an der Endplatte 6 des Wirbelkörpers 1 und mit seiner Unterseite an der Endplatte 7 des Wirbelkörpers 2 ab, die Rippen 21 und 22 greifen in die Nuten 18 bzw. 19 ein und legen dadurch das Implantat 20 in dieser Position fest.

Das Implantat 20 besteht aus einem resorbierbaren Material und ist porös ausgebildet, die Größe der Poren liegt zwischen 150 und 200 µm. Das Material des stopfenförmigen Implantates 20 ist flexibel und elastisch, so daß es sich optimal an die Kontur der Öffnung 8 anpaßt und auch die Bewegungen des Materials des Annulus fibrosus ohne weiteres mitmacht. Dagegen sind die Rippen 21 und 22 formstabil ausgebildet, also wesentlich härter und nicht in gleicher Weise verformbar oder elastisch wie das übrige Material des Implantates 20. Dadurch ergibt sich im Bereich der Festlegung des Implantates 20 an den benachbarten Wirbelkörpern 1, 2 eine formstabile Verbindung, dazwischen jedoch paßt sich das Material des Implantates 20 an die Beweglichkeit des Materials des Annulus fibrosus an und verschließt die Öffnung 8 zuverlässig.

Das gesamte Material des Stopfens zwischen den Rippen 21, 22 kann in dieser Weise flexibel oder elastisch ausgebildet sein, es ist aber auch möglich, daß das Material des Stopfens zu den Rippen 21, 22 hin zunehmend fester wird, beispielsweise könnte die Oberseite und die Unterseite des Stopfens in ähnlicher Weise formstabil ausgebildet sein wie die Rippen 21, 22, so daß sie formstabile Anlageflächen an die Endplatten der Wirbelkörper ausbilden. Wichtig ist lediglich, daß das Material des Stopfens im mittleren Bereich flexibel und elastisch ist, um eine Anpassung an die Beweglichkeit des Annulus-fibrosus-Gewebes zu gewährleisten.

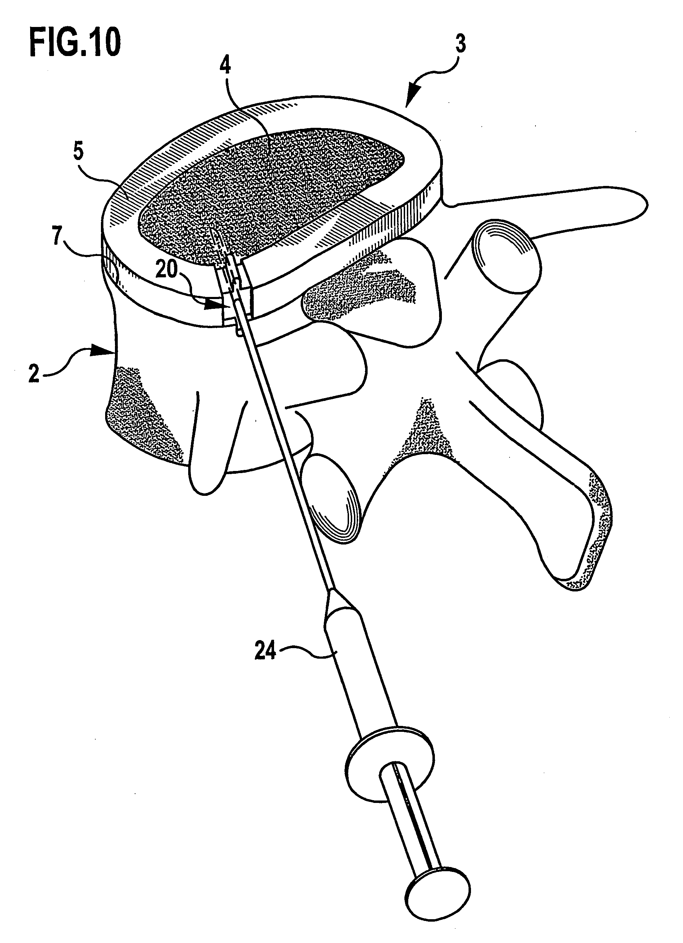

Wenn die Öffnung 8 im Annulus fibrosus 5 auf diese Weise verschlossen ist, kann man das außerhalb des Körpers angezüchtete Zellmaterial in das Innere der Bandscheibe 3 einfüllen. Dies erfolgt in der Regel etwa zwei Wochen nach der vorstehend beschriebenen Operation. Die Einfüllung kann mit Hilfe einer Injektionsnadel 24 erfolgen (Figur 10), die durch das Implantat 20 hindurch in den Innenraum der Bandscheibe 3 eingeführt wird und das Material sowohl in diesen Innenraum als auch in den porösen Bereich des Implantates 20 injiziert. Die injizierten Körperzellen wachsen im Innenraum der Bandscheibe 3 und im Implantat 20 an und verschließen schließlich die Öffnung 8 zuverlässig, so daß die Öffnung auch dann verschlossen bleibt, wenn sich das Implantat 20 allmählich zersetzt und vom Körper resorbiert wird.

Bei dem in den Figuren 1 bis 10 dargestellten Ausführungsbeispiel ist das Implantat 20 im wesentlichen würfelförmig oder quaderförmig ausgebildet und trägt an seiner Oberseite und an seiner Unterseite jeweils einen mittig angeordneten Steg.

Bei dem abgewandelten Ausführungsbeispiel der Figur 3 ist das Implantat 20 auch im wesentlichen quaderförmig ausgebildet, jedoch breiter, und dementsprechend sind auf der Oberseite und der Unterseite zwei parallel zueinander verlaufende Rippen 21 bzw. 22 angeordnet, die von den Seitenflächen des Implantates 20 einen Abstand einhalten, so daß im Bereich der Seitenflächen das Implantat aufgrund der nachgiebigen und elastischen Struktur sich optimal an das Gewebe des Annulus fibrosus anpassen kann, während das Implantat 20 durch die parallel verlaufenden Rippen 21, 22 in entsprechenden doppelten Nuten 18, 19 in den Endplatten der Wirbelkörper fixiert wird.

Anstelle der Rippen 21, 22 könnten auch mehrere stiftförmige Vorsprünge vorgesehen werden, die in einer Reihe nebeneinander liegen. Außerdem ist es zur besseren Festlegung des Implantates in den Wirbelkörpern möglich, die Rippen oder Vorsprünge mit einer Zahnung zu versehen, die in die Seitenwände der Nuten 18, 19 eingreifen.

An seiner Außenseite kann das Implantat 20 mit einer Membran 25 abgedeckt sein (Figur 1b), die dicht ausgebildet ist und das poröse Implantat 20 nach außen hin abdichtet. Auch diese Membran 25 kann aus resorbierbarem Material bestehen, vorzugsweise zersetzt sich dieses Material langsamer als das Material des übrigen Implantates 20 und wird dementsprechend später resorbiert.

Außerdem kann in diesem Bereich eine in der Zeichnung nicht näher dargestellte Anti-Adhäsionsschicht aufgetragen werden, diese kann beispielsweise aus einem gelatinehaltigen Gel oder Spray bestehen. Dadurch kann eine postoperative Entstehung von Narbengewebe verhindert werden.

## Patentansprüche

1. Implantat zum Verschluß einer Öffnung (8) des Annulus fibrosus (5) in Form eines Stopfens (20), **dadurch gekennzeichnet, daß** der Stopfen (20) an seiner Oberseite und an seiner Unterseite formstabile Verankerungselemente (21, 22) für die Endplatten (6, 7) benachbarter Wirbelkörper (1, 2) und im Bereich zwischen Oberseite und Unterseite eine verformbare Struktur aufweist

2. Implantat nach Anspruch 1, **dadurch gekennzeichnet, daß** die Oberseite und/oder die Unterseite formstabiler sind als die zwischen Oberseite und Unterseite liegenden Bereiche des Stopfens (20).

3. Implantat nach Anspruch 1 oder 2, **dadurch gekennzeichnet, daß** es aus resorbierbarem Material besteht.

4. Implantat nach einem der voranstehenden Ansprüche, **dadurch gekennzeichnet, daß** der Stopfen (20) aus porösem Material besteht.

5. Implantat nach Anspruch 4, **dadurch gekennzeichnet, daß** die Porengröße des porösen Materials zwischen 150 µm und 200 µm liegt.

6. Implantat nach einem der voranstehenden Ansprüche, **dadurch gekennzeichnet, daß** die Oberseite und die Unterseite mindestens einen in die Endplatte (6, 7) des benachbarten Wirbelkörpers (1, 2) einsetzbaren Vorsprung (21, 22) tragen.

7. Implantat nach Anspruch 6, **dadurch gekennzeichnet, daß** mehrere Vorsprünge in einer Linie nebeneinander liegen.

8. Implantat nach Anspruch 6, **dadurch gekennzeichnet, daß** der Vorsprung eine Längsrippe (21, 22) ist.

9. Implantat nach einem der Ansprüche 7 oder 8, **dadurch gekennzeichnet, daß** die Linie der Vorsprünge bzw. die Längsrichtung der Längsrippen (21, 22) parallel zur Einschubrichtung des Stopfens (20) verläuft.

10. Implantat nach einem der Ansprüche 6 bis 9, **dadurch gekennzeichnet, daß** der Vorsprung (21, 22) eine Zahnung aufweist.

11. Implantat nach einem der Ansprüche 6 bis 10, **dadurch gekennzeichnet, daß** quer zur Einschubrichtung des Stopfens (20) nebeneinander mehrere Vorsprünge (21, 22) angeordnet sind.

12. Implantat nach Anspruch 11, **dadurch gekennzeichnet, daß** die Vorsprünge (21, 22) gegenüber der Seitenfläche des Stopfens (20) zurückgesetzt sind.

13. Implantat nach einem der voranstehenden Ansprüche, **dadurch gekennzeichnet, daß** der Stopfen (20) die Form eines Würfels oder Quaders hat.

14. Implantat nach einem der Ansprüche 3 bis 13, **dadurch gekennzeichnet, daß** der Stopfen (20) mit einer Membran (25) abgedeckt ist, die ebenfalls aus resorbierbarem Material besteht.

15. Implantat nach Anspruch 14, **dadurch gekennzeichnet, daß** die Membran (25) langsamer resorbierbar ist als der Stopfen (20).

16. Implantat nach einem der voranstehenden Ansprüche, **dadurch gekennzeichnet, daß** der Stopfen (20) an seiner Außenseite mit einem Anti-Adhäsionsmittel versehen ist.

17. Implantat nach einem der voranstehenden Ansprüche, **dadurch gekennzeichnet, daß** es Teil eines Satzes von Stopfen (20) mit unterschiedlicher Höhe und/oder Breite ist.

## Claims

1. Implant in the form of a plug (20) for closing an opening (8) in the annulus fibrosus (5), **characterized in that** the plug (20) has rigid anchoring elements (21, 22) for the end plates (6, 7) of adjacent vertebrae (1, 2) on its upper face and on its lower face, and a deformable structure in the area between upper face and lower face.

2. Implant in accordance with claim 1, **characterized in that** the upper face and/or the lower face are more rigid than the areas of the plug (20) lying between upper face and lower face thereof.

3. Implant in accordance with claim 1 or 2, **characterized in that** it consists of resorbable material.

4. Implant in accordance with any one of the preceding claims, **characterized in that** the plug (20) consists of porous material.

5. Implant in accordance with claim 4, **characterized in that** the pore size of the porous material lies between 150 µm and 200 µm.

6. Implant in accordance with any one of the preceding claims, **characterized in that** the upper face and the lower face carry at least one projection (21, 22) insertable in the end plate (6, 7) of the adjacent vertebra (1, 2).

7. Implant in accordance with claim 6, **characterized in that** several projections lie alongside one another in a line.

8. Implant in accordance with claim 6, **characterized in that** the projection is a longitudinal rib (21, 22).

9. Implant in accordance with claim 7 or 8, **characterized in that** the line of the projections or the longitudinal direction of the longitudinal ribs (21, 22) extends parallel to the direction of insertion of the plug (20).

10. Implant in accordance with any one of claims 6 to 9, **characterized in that** the projection (21, 22) has a toothing.

11. Implant in accordance with any one of claims 6 to 10, **characterized in that** several projections (21, 22) are arranged alongside one another transversely to the direction of insertion of the plug (20).

12. Implant in accordance with claim 11, **characterized in that** the projections (21, 22) are set back from the side surface of the plug (20).

13. Implant in accordance with any one of the preceding claims, **characterized in that** the plug (20) has the shape of a cube or a rectangular parallelepiped.

14. Implant in accordance with any one of claims 3 to 13, **characterized in that** the plug (20) is covered with a membrane (25) which also consists of resorbable material.

15. Implant in accordance with claim 14, **characterized in that** the membrane (25) is resorbable more slowly than the plug (20).

16. Implant in accordance with any one of the preceding claims, **characterized in that** the plug (20) is provided with an anti-adhesion agent on the outer side thereof.

17. Implant in accordance with any one of the preceding claims, **characterized in that** it is part of a set of plugs (20) having a different height and/or width.

## Revendications

1. Implant destiné à fermer une ouverture (8) de l'anneau fibreux (5) sous la forme d'un tampon (20), **caractérisé en ce que** le tampon (20) présente sur sa face supérieure et sur sa face inférieure des éléments d'ancrage indéformables (21, 22) pour les plaques d'extrémité (6, 7) de vertèbres adjacentes (1, 2) et une structure déformable dans la zone entre la face supérieure et la face inférieure.

2. Implant selon la revendication 1, **caractérisé en ce que** la face supérieure et/ou la face inférieure sont plus de forme plus stable que les zones du tampon (20) situées entre la face supérieure et la face inférieure.

3. Implant selon la revendication 1 ou 2, **caractérisé en ce qu'**il se compose d'un matériau résorbable.

4. Implant selon l'une quelconque des revendications précédentes, **caractérisé en ce que** le tampon (20) se compose d'un matériau poreux.

5. Implant selon la revendication 4, **caractérisé en ce que** la dimension des pores du matériau poreux se situe entre 150 µm et 200 µm.

6. Implant selon l'une quelconque des revendications précédentes, **caractérisé en ce que** la face supérieure et la face inférieure portent au moins une saillie (21, 22) pouvant être insérée dans la plaque d'extrémité (6, 7) de la vertèbre adjacente (1, 2).

7. Implant selon la revendication 6, **caractérisé en ce que** plusieurs saillies se trouvent côte à côte sur une ligne.

8. Implant selon la revendication 6, **caractérisé en ce que** la saillie est une nervure longitudinale (21,22).

9. Implant selon l'une quelconque des revendications 7 ou 8, **caractérisé en ce que** la ligne des saillies ou la direction longitudinale des nervures longitudinales (21, 22) s'étend en parallèle à la direction d'insertion du tampon (20).

10. Implant selon l'une quelconque des revendications 6 à 9, **caractérisé en ce que** la saillie (21, 22) présente une denture.

11. Implant selon l'une quelconque des revendications 6 à 10, **caractérisé en ce que** transversalement à la direction d'insertion du tampon (20), plusieurs saillies (21, 22) sont disposées côte à côte.

12. Implant selon la revendication 11, **caractérisé en ce que** les saillies (21, 22) sont enfoncées par rapport à la surface latérale du tampon (20).

13. Implant selon l'une quelconque des revendications précédentes, **caractérisé en ce que** le tampon (20) présente la forme d'un cube ou d'un parallélépipède.

14. Implant selon l'une quelconque des revendications 3 à 13, **caractérisé en ce que** le tampon (20) est recouvert d'une membrane (25) qui se compose également d'un matériau résorbable.

15. Implant selon la revendication 14, **caractérisé en ce que** la membrane (25) est plus lentement résorbable que le tampon (20).

16. Implant selon l'une quelconque des revendications précédentes, **caractérisé en ce que** le tampon (20) est muni sur sa face extérieure d'un moyen antiadhésif.

17. Implant selon l'une quelconque des revendications précédentes, **caractérisé en ce qu'**il fait partie d'un jeu de tampons (20) de hauteur et/ou largeur différente(s).
